# EUROPEAN PATENT APPLICATION

(11) **EP 3 404 409 A1**
(43) Date of publication of application: **21.11.2018**
(21) Application number: 17171883.6
(22) Date of filing: 19.05.2017
(51) Int. Cl.: G01N 33/02, G01N 22/00

(54) **METHOD AND SYSTEM FOR EVALUATING FOOD SAFETY**

(71) Applicant: Nederlandse Organisatie voor toegepast- natuurwetenschappelijk onderzoek TNO, 2595 DA 's-Gravenhage (NL)
(72) Inventor: The designation of the inventor has not yet been filed
(74) Representative: V.O.

(57) **Abstract**

A method and system for evaluating food safety of a food product (P). An electric signal (Se) is used for generating an oscillating electric field (E) in a radio wave frequency range permeating at least part of the food product (P) and measuring a response of the permeated part of the food product (P) to the electric field (E) to determine a test value (e2) indicative of a permittivity (ε) of the food product (P) at one or more frequencies (ω) in the radio wave frequency range. The test value (e2) is compared with a reference value (e1) indicative of the permittivity (ε) at the at the same one or more frequencies (ω) of a reference food product (P') in a known coagulation state (U). An evaluation parameter can be generated for rejecting (R) or accepting (A) the food product (P) based on the comparison.

## Description

### TECHNICAL FIELD AND BACKGROUND

The present disclosure relates to the processing of food products and particularly to methods and systems for evaluating food safety.

Methods of food processing may involve steps to reduce harmful micro-organisms and safety check to determine the likelihood of their occurrence. For example, food is processed by heating to a temperature deemed sufficient for sterilization. However, because the exact sterilization temperature may be difficult to determine, the outside temperature may be raised unnecessarily high. This can lead to energy or product waste. Moreover, temperature inside the product may be difficult to determine without inserting a thermometer that damages the product and may not represent different parts of the product (point measurement).

The prior art CN 103237078 A relates to a near infrared food safety authentication system comprising a sampling probe used to collect the near-infrared spectral data of tested food. A networked terminal connects the sampling probe to transmit the near infrared spectral data to an authentication center system comprising a food fingerprint spectrum library. The library identifies the near infrared spectrum to identify the safety level of the measured food as reflected by the near infrared spectrum data and transmit it to the networked terminal. Unfortunately, infrared measurement may involve complicated equipment such as light sources and spectrometers. Also the infrared spectra may not be suitable for detecting some relevant food qualities, especially inside the food product.

Improved methods and systems for evaluating food safety are thus desired. Particular interest may exist for providing subsurface measurements which better correlate with food safety and which can be easily performed on a mass scale without damaging the products, or even without contacting the products.

### SUMMARY

Some aspects of the present disclosure may relate to methods for evaluating a food product. An electric signal may be used for generating an oscillating electric field. The electric field preferably oscillates in a radio frequency (RF) range and permeates at least part of the food product under test. A response of the permeated part of the food product to the electric field can be measured to determine a test value. The test value is indicative of a permittivity of the food product at one or more frequencies in the radio frequency range. The test value can be compared with a reference value. For example, the reference value is indicative of the permittivity at the same one or more frequencies of a reference food product in a known degree of coagulation, e.g. fully or partially coagulated or uncoagulated.

A difference in the test values can thus be indicative of a difference in the permittivity of the tested food product and reference food product, which difference in permittivity is indicative of a difference in the coagulations states of the tested food product and reference food product. It is currently recognized that similar conditions responsible for changing the coagulation state of the food product may also effect the neutralization of undesired micro-organisms in the food product, e.g. killing them by denaturation. Accordingly, a comparison of the test value can be used to evaluate food safety of the food product. The generation and measurement of RF electric fields can be relatively simple e.g. using a signal generator with contacting electrical wires or even contactless using one or more antennas. Accordingly, the evaluation of food safety can be easily performed on a mass scale without damaging the products, or even without contacting the products

Various applications can be envisaged for meat and other food products as described herein. For example, a first permittivity is measured -as reference- while a food product is known to be raw, i.e., in an uncoagulated state at its initial temperature, e.g., room temperature. The same (or another similar) food product is heated to an elevated temperature which may change the coagulation state. This may happen if the temperature in the food product is high enough and/or applied for a sufficient amount of time. To test whether this condition has indeed occurred, a second permittivity is measured after the heating, preferably with the product back to its initial temperature. If the heating was sufficient to change the coagulation state, this structural change can have a substantial effect on the permittivity. Accordingly the second permittivity of the cooked product may be significantly different from the first permittivity of the raw product. This expected difference can be used to evaluate if the product was cooked to sufficiently change its coagulation state and, correspondingly, whether undesired micro-organisms in the food product were sufficiently neutralized. If the permittivity of the food product did not sufficiently change as a result of the heating, this may indicate that its structure has not substantially changed, i.e., it is still raw.

Further aspects of the present disclosure may relate to a system for evaluating food safety of a food product, e.g., as part of a processing plant. The system typically comprises a support surface such as a conveyor belt configured to support the food product while it is processed and/or evaluated. As described herein, a signal generator may be configured to generate an electric signal in a radio wave frequency range. A product probe may be arranged in proximity to the product support surface. The probe can be configured to receive the electric signal and generate an oscillating electric field. In use, the electric field may permeate at least part of the food product. The same or different probe can be used to measure a response of the permeated part of the food product to the electric field, e.g., measure a resulting electric field. This can be used to determine a test value indicative of a permittivity of the food product at one or more frequencies in the radio wave frequency range. Optionally, a memory can be used to store a reference value indicative of the permittivity of a reference food product in a known coagulation state. A comparator (circuit or software) can be configured to compare the test value with the reference value. Optionally, an evaluation module can be configured to generate an evaluation parameter for rejecting or accepting the food product based on the comparison. The system may be equipped with (additional) hardware and/or programmed with software instructions that, when executed, cause the system to perform operational acts as described herein.

### BRIEF DESCRIPTION OF DRAWINGS

These and other features, aspects, and advantages of the apparatus, systems and methods of the present disclosure will become better understood from the following description, appended claims, and accompanying drawing wherein:
FIGs 1A-1C schematically illustrate steps for evaluating food safety according to some embodiments;
FIGs 2A-2C schematically illustrate embodiments of various product probes;
FIGs 3A and 3B schematically illustrate different systems for evaluating food safety according to some embodiments;
FIG 4A shows a picture of a test setup for evaluating food safety;
FIG 4B shows measured spectra of the relative permittivity as a function of frequency for different food products
FIGs 5A and 5B schematically illustrate graphs for evaluating food safety according to some embodiments;

### DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise. The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

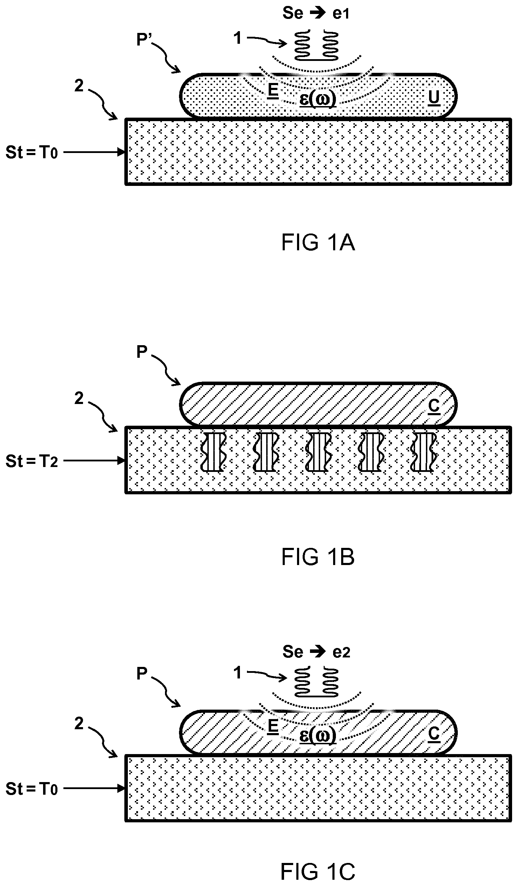

FIGs 1A-1C schematically illustrate steps for evaluating food safety according to some embodiments.

FIG 1A illustrates using an electric signal Se for generating an electric field E. The electric field E preferably oscillates in a radio wave frequency range permeating at least part of the reference food product P'. For example, one or more frequencies of the electric field can be selected having a desired penetration depth in the food product. A response can be measured including that of the permeated part of the reference food product P' to the electric field E. The response can be used to determine a reference value e1 indicative of a permittivity ε of the reference food product P' at one or more frequencies ω in the radio wave frequency range.

FIG 1B illustrates a step of processing the food for changing its coagulation state from an initial uncoagulated state U to a coagulated state C. In the present embodiment, the coagulation state is changed by increasing the temperature from an initial temperature T0 to an elevated temperature T2. For example the temperature is set by changing a temperature signal St to a heater 2.

FIG 1C illustrates a similar step as FIG 1A, but now after the food is processed. In this step, the food product P is tested to determine the food safety. The same or similar electric signal Se is used for generating an electric field E in the food product P. A response is measured to determine a test value e2 indicative of a permittivity ε of the food product P under test at one or more frequencies ω in the radio wave frequency range. In a preferred embodiment, the test value e2 is compared with the reference value e1 of the reference food product P' in the known coagulation state, which in this case was uncoagulated U. Typically, a difference in the test values e1 and e2 is indicative of a difference in the permittivity ε of the tested food product P and reference food product P', which difference in permittivity ε is indicative of a difference in the coagulations states C,U of the tested food product P and reference food product P'. In some embodiments, an evaluation parameter is generated, e.g., for rejecting or accepting the food product P based on the comparison.

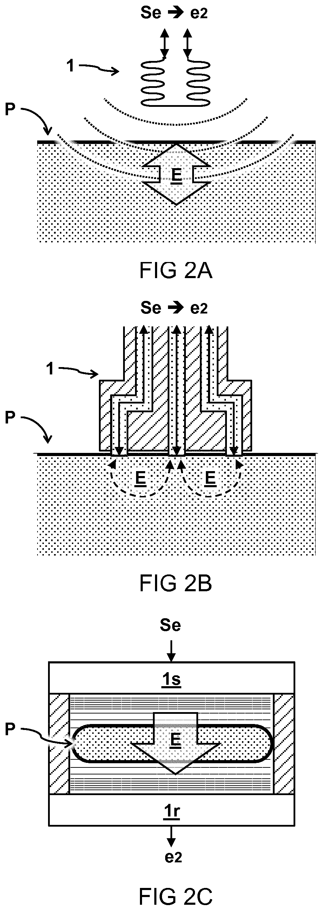

FIGs 2A-2C schematically illustrate embodiments of various product probes 1 for transmitting and/or receiving electric signals and fields.

FIG 2A schematically illustrates a preferred embodiment wherein the electric field E is generated by a contactless product probe 1, e.g., one or more antennas for emitting and/or receiving radio waves. In the embodiment shown, one antenna acts as transceiver in proximity to the food product P, wherein the electric field E permeating the food product P is at least partially reflected. In another embodiment (not shown), multiple antennas are used for receiving a response, e.g., electrical field interacting with the food product P. For example, the antennas are arranged in a one or two-dimensional array.

FIG 2B illustrates another embodiment wherein the electric field E is generated by a product probe 1 contacting the food product P, e.g., a coax cable with a contacting element that can be pressed against a surface of the food product P. It will be appreciated that the contacting probe does not need to penetrate the food product P, e.g., unlike a thermometer. The electric field can permeate into the food product P without damage.

FIG 2C illustrates yet another embodiment wherein the food product P is disposed in a path of the electric field E between two probes, e.g., a sending probe Is on one side and a receiving prove on the other side. In the embodiment shown, the probes may act as antennas, e.g., sending the electric field E through a waveguide comprising the food product P. Also contacting probes 1s and 1r may be envisaged, e.g., contacting electrodes on either sides. Instead of dedicated transmitter and receiver probes, the probes may also act as transceivers, both sending and receiving electric fields.

In one embodiment (not shown), the measurement is performed using radar. For example, a phased array antenna and/or synthetic aperture radar. In some embodiments, a single sender antenna generates an oscillating electrical field which is picked up by multiple receiver antennas after interaction with the food product P. One advantage of using multiple antennas may be an increased coverage and/or resolution. For example, an RF system, using multiple antennas in a coherent manner, using motion of the objects, and/or using modulation of the signal, can get a relatively high spatial resolution in 3D while maintaining a large coverage (e.g., total width of the conveyer belt). Various signal processing techniques can also be used to obtain the information.

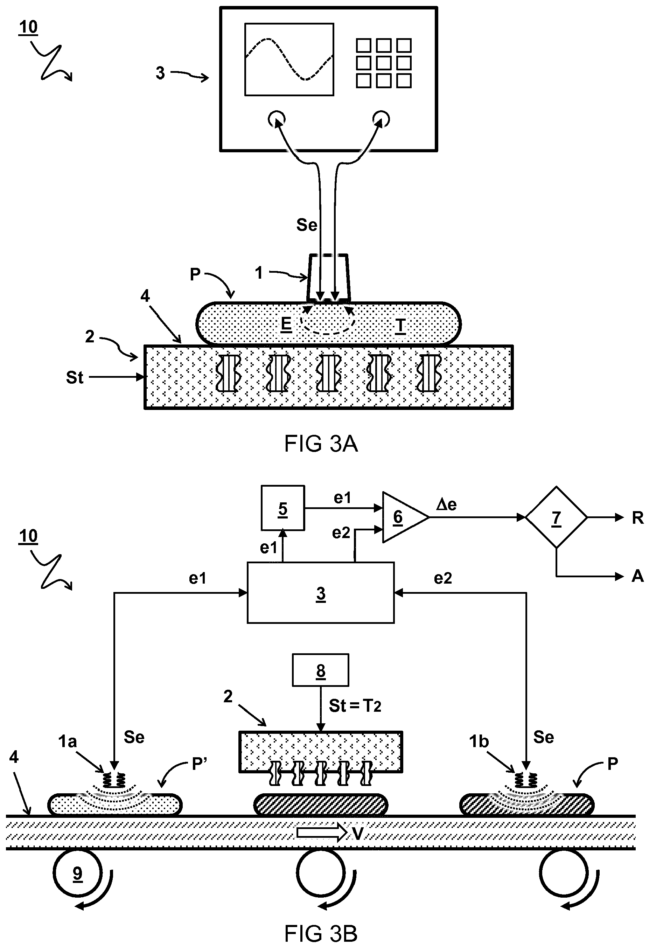

FIGs 3A and 3B schematically illustrate different systems 10 for evaluating food safety according to some embodiments.

Typically, a system 10 for evaluating food safety of a food product P comprises a support surface 4 configured to support the food product P while it is evaluated. In the embodiment of FIG 3A the food product P is on a support surface 4 of a heater 2. In the embodiment of FIG 3B, the food product P moves on the support surface 4 of a conveyor belt. Aspects of the embodiments can also be combined.

In one embodiment, a signal generator 3 configured to generate an electric signal Se, preferably in a radio wave frequency range. The same or different device can also be used for measuring the response values e1 and e2. In other or further embodiments, at least one product probe 1 arranged in proximity to the support surface 4 (i.e., near the food product P to be placed thereon). The product probe 1 is configured to receive the electric signal Se and generate an oscillating electric field E permeating at least part of the food product P. A response is measured of the permeated part of the food product P to the electric field E to determine a test value e2 as described herein.

In one embodiment, a storage medium such as a memory 5 is used for storing a reference value e1 indicative of the permittivity of a reference food product P' in a known coagulation state, e.g., coagulated or uncoagulated. For example, the reference value e1 is based on one or more previous measurements on the same or similar food products. In some embodiments, the memory 5 stores multiple reference values e1 indicative of the permittivity ε of reference food products P' in different coagulation states or different degrees of coagulation. For example, a database is used to store reference values or spectra corresponding to different degrees of coagulation. The reference values may be also be recorded and/or stored as function of different parameters, e.g., temperature of the food product P or different food products.

In some embodiments, such as FIG 3B, the support surface 4 comprises a moving surface configured to transport the food product P. For example, a velocity V of the support surface 4 is set by a transport mechanism 9. In the embodiment shown, the support surface 4 is configured to move the food product P between a first product probe 1a configured to measure the reference value e1 and a second product probe 1b configured to measure the test value e2. In some embodiments, the system 10 comprises a heater 2 for heating the food product P. In the embodiment shown, a temperature T2 of the heater 2 is set by a temperature controller 8. For example, the temperature is set St=T2 for changing the coagulation state of the food product P. In a preferred embodiment, the heater 2 is disposed in a product path between the measurement of the reference value e1 and the measurement of the test value e2, e.g. between the first product probe 1a and the second product probe 1b.

In one embodiment, a comparator 6 is configured to compare the test value e2 with the reference value e1. In another or further embodiment, an evaluation module 7 is configured to generate an evaluation parameter for rejecting (R) or accepting (A) the food product P based on the comparison. In another or further embodiment, the evaluation parameter R,A is used for controlling process parameters for processing the food product P. In some embodiments, the evaluation parameter is used for a temperature of the temperature controller 8 and/or velocity of the transport mechanism 9. For example, the temperature is increased and/or the velocity decreased if the tested product P is insufficiently coagulated. For example, the temperature is decreased and/or the velocity increased if the tested product P is coagulated too much. In some embodiments, the system 10 comprises a discarding mechanism (not shown here). For example, the discarding mechanism may be configured to receive the evaluation parameter R,A and discard the food product P when it is rejected (R) and/or allow the product to proceed further when it is accepted (A).

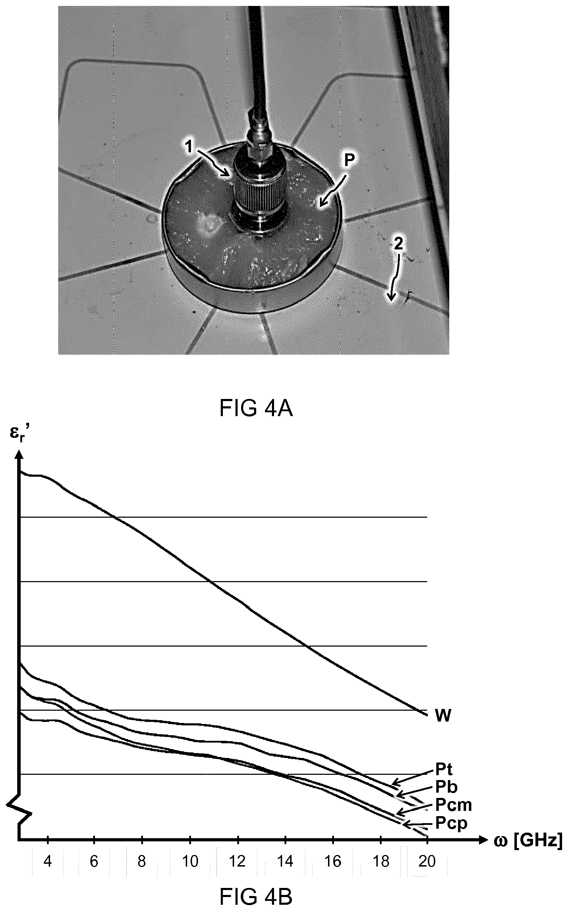

FIG 4A shows a picture of a test setup for evaluating food safety of a food product P (in this case raw chicken meat). In the setup, the product P is placed in a container on top of a heater 2. A probe 1 in this case is formed by a coax cable with its center and surrounding electrodes contacting the product.

FIG 4B shows measured spectra of the relative permittivity εᵣ' as a function of frequency ω for different tested products. The spectrum of turkey meat is indicated by "Pt", beef is indicated by "Pb", chicken meat is indicated by "Pcm", chicken paste is indicated by "Pcp". For reference, also a spectrum of water is indicated by "W".

In a preferred embodiment, the electric signal Se is generated at one or more frequencies in a radio wave frequency range, e.g., between 3 kHz - 300 GHz, preferably 1 MHz- 50 GHz, more preferably 0.1-20 GHz. Lower frequencies, e.g., below 1 GHz or in a MHz range, may have an advantage of higher penetration. Higher frequencies, e.g., above 1 GHz may have an advantage of sampling a more specific location. In some embodiments, the test value and reference value are based on measurement of the permittivity at multiple frequencies.

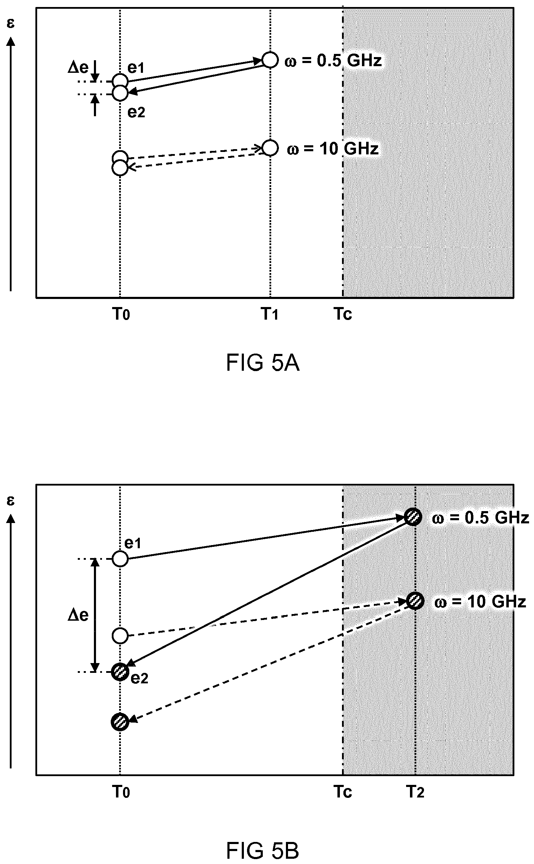

FIGs 5A and 5B schematically illustrate graphs for evaluating food safety according to some embodiments. The figures illustrate typical graph of the permittivity ε at specific frequencies ω (0.5 and 10 GHz). The permittivity is measured as function of temperature according to a specific heating cycle or trajectory.

FIG 5A illustrates a typical cycle wherein the product is heated to a first temperature T1 that is below the coagulation temperature Tc. Typically, such heating cycle does not substantially change the permittivity ε, at least when the product is brought back to its initial temperature T0. There may be some small difference Δe between the initial and final values e1,e2, e.g. caused by evaporation of water.

FIG 5B illustrates a typical cycle wherein the product is heated to a second temperature T2 that is above the coagulation temperature Tc. This may change the state from being initially uncoagulated (indicated by white marker) to coagulated (indicated by hashed marker). The change of structure may be measurable by a more substantial difference Δe between the initial and final values e1,e2 than in the previous case.

In some embodiments, food product as described herein may comprise meat. For example, the food product P' in the uncoagulated state U comprises raw meat. For example, the food product P' in the coagulated state C comprises cooked meat. Preferably the electric field E is applied to a piece of the meat without bone. Alternative to meat also other food products may be tested for coagulation or other state changes using test values indicating the permittivity. For example, products such as eggs, bread, or other baked products may undergo state changes after heating.

Typically, individual protein molecules in uncooked food products such as raw meat, are wound-up in coils which are formed and held together by bonds. When the food product is heated, the bonds can break and the protein molecule unwinds. Heat may also shrink muscle fibers both in diameter and in length as water is squeezed out and the protein molecules recombine, or coagulate. As coagulation occurs amino acid chains may undergo permanent changes or denaturation. For example, peptide bonds are broken causing the amino acid chains to unravel. Similar processes can also contribute to neutralizing undesired micro-organisms. It is currently found that the structural changes associated with coagulation or denaturing of the food product can be related to changes in the material permittivity.

Permittivity is a measure of resistance that is encountered when forming an electric field in a particular medium (the food product), e.g., at a particular (radio wave) frequency. The permittivity of a dielectric medium can, e.g., be represented by the ratio of its absolute permittivity to the electric constant or vacuum permittivity (ε0). This dimensionless quantity is also called the medium's relative permittivity (εᵣ) or dielectric constant. Generally, the relative permittivity may be a complex valued quantity, dependent on frequency. For example, a quantity of interest may be the real part of the medium's relative permittivity at a particular frequency εᵣ'(ω). Alternatively, or in addition, the imaginary part, absolute value, or phase of the permittivity may provide an equivalent or additional measure of interest. The permittivity can also be directly or indirectly related to other material quantities such as the electric susceptibility (χe). It will thus be understood that the permittivity of the food product, as measured herein, may be represented in different forms, e.g., scaling or other transformation, while remaining indicative of the material properties under investigation, in particular its coagulation state. For example, the test value indicates a reaction (e.g., resistance) of the material to an oscillating electric field permeating the material.

In some embodiments, the food product is rejected if its test value e2 corresponds to an uncoagulated state U or does not correspond to a coagulated state C. For example, the food product P is rejected if the difference in permittivity related value Δe compared to a reference uncoagulated state U is below a first threshold difference and/or the food product P is rejected if the difference in permittivity related value Δe compared to a reference coagulated state C is above a second threshold difference.

In other or further embodiments, the food product is accepted if its test value e2 indicates it is in a coagulated state C or not in an uncoagulated state U. For example, the food product P is accepted if the difference in permittivity related value Δe compared to a reference uncoagulated state C is above a first threshold difference and/or the food product P is accepted if the difference in permittivity related value Δe compared to a reference coagulated state C is below a threshold difference.

In some embodiments, the reference food product P' is the same as the food product P under test, but at an earlier time with the reference value e1 being measured at that earlier time. Alternatively the reference value e1 is based on one or more reference products, e.g., previously stored values of similar products in known coagulation states, e.g., coagulated, uncoagulated, or possible intermediate states with a certain degree of coagulation. In other or further embodiments, the evaluation comprises fitting a spectrum of the permittivity ε to one of multiple spectra having different known coagulation states, and determining the coagulation state of the product under test based on the known state corresponding to the best fitting spectrum of one of the reference products. Preferably, one or more reference products P' are at least in the same product class as the food product P under test, e.g., one or more of the same animal type, same part of the animal, same consistency (ground, paste), same preparation [cooking, grill, seasoning], same size, thickness, et cetera.

In some embodiments, the food product P is heated from an initial temperature to an elevated temperature T2 for changing its coagulation state, normally from an initial uncoagulated state U to a final coagulated state C. Preferably, the permittivity related test value e2 of the food product P is measured at similar circumstances as the permittivity related reference value e1. For example, the test value e2 is measured using the same electric signal Se as the reference value e1. Preferably, the test value e2 of the food product P is measured at the same temperature T0 as the reference value e1, or a similar temperature, e.g., within twenty, ten, or five degrees Celsius. For example, the food product P is brought back to the initial temperature T0 before measuring the second permittivity e2 to account for any temperature dependent differences.

Some embodiments comprise measuring a first value e1 as reference indicative of the permittivity ε of the food product P' known to be initially in an uncoagulated state U; heating the food product P'; and measuring a second value e2 as test of the permittivity ε of the food product P in an unknown state to determine if the product has changed from the initial uncoagulated state U to a coagulated state C by the heating. In other or further embodiments, the food product P is rejected or the heating is repeated or continued if the food product P is still in the uncoagulated state U. In one embodiment, the test value e2 is continuously or periodically measured while heating the food product P. In another or further embodiment, the heating is controlled based on a continuously or periodically measured test value e2. For example, heating the food product P is stopped upon determination that the test value e2 sufficiently differs from a reference value e1.

For the purpose of clarity and a concise description, features are described herein as part of the same or separate embodiments, however, it will be appreciated that the scope of the invention may include embodiments having combinations of all or some of the features described. For example, while embodiments were shown for contactless probes may also employ contacting probes, waveguides et cetera. While heating is shown as a method for changing the coagulation state also other processes may be used. Instead of or alternative to changing the coagulation state also other structural changes may be tested by the methods as described herein. The various elements of the embodiments as discussed and shown offer certain advantages, such as testing food safety. Of course, it is to be appreciated that any one of the above embodiments or processes may be combined with one or more other embodiments or processes to provide even further improvements in finding and matching designs and advantages. It is appreciated that this disclosure offers particular advantages to food processing, and in general can be applied for any application to test circumstances correlated with specific structural changes in products.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. Where one claim refers to another claim, this may indicate synergetic advantage achieved by the combination of their respective features. But the mere fact that certain measures are recited in mutually different claims does not indicate that a combination of these measures cannot also be used to advantage. The present embodiments may thus include all working combinations of the claims wherein each claim can in principle refer to any preceding claim unless clearly excluded by context.

## Claims

1. A method for evaluating food safety of a food product (P), the method comprising
- using an electric signal (Se) for generating an oscillating electric field (E) in a radio wave frequency range permeating at least part of the food product (P) and measuring a response to determine a test value (e2) indicative of a permittivity (ε) of the food product (P) at one or more frequencies (ω) in the radio wave frequency range;
- comparing the test value (e2) with a reference value (e1) indicative of the permittivity (ε) of a reference food product (P') in a known coagulation state (U); and
- generating an evaluation parameter for rejecting (R) or accepting (A) the food product (P) based on the comparison.

2. The method according to claim 1, wherein the food product is rejected if its test value (e2) corresponds to an uncoagulated state (U) or does not correspond to a coagulated state (C).

3. The method according to any of the preceding claims, wherein the reference food product (P') is the same as the food product (P) under test, but at an earlier time with the reference value (e1) being measured at that earlier time.

4. The method according to any of the preceding claims, wherein the food product (P) is heated from an initial temperature (T0) to an elevated temperature (T2) for changing its coagulation state.

5. The method according to any of the preceding claims, wherein the test value (e2) is measured using the same electric signal (Se) as the reference value (e1).

6. The method according to any of the preceding claims, comprising
- measuring a first value (e1) as reference indicative of the permittivity (ε) of the food product (P') known to be initially in an uncoagulated state (U);
- heating the food product (P'); and
- measuring a second value (e2) as test of the permittivity (ε) of the food product (P) in an unknown state to determine if the product has changed from the initial uncoagulated state (U) to a coagulated state (C) by the heating.

7. The method according to any of the preceding claims, wherein the electric signal (Se) is generated at one or more frequencies in a radio wave frequency range between 3 kHz - 300 GHz.

8. The method according to any of the preceding claims, wherein the test value (e2) and reference value (e1) are based on measurement of the permittivity (ε) at multiple frequencies.

9. The method according to any of the preceding claims, wherein the electric field (E) is generated by a contactless product probe (1).

10. The method according to any of the preceding claims, wherein the response is measured by an antenna array.

11. The method according to any of the preceding claims, wherein the food product (P) comprises meat.

12. A system (10) for evaluating food safety of a food product (P), the system comprising
- a support surface (4) configured to support the food product (P) while it is evaluated;
- a signal generator (3) configured to generate an electric signal (Se) in a radio wave frequency range;
- a product probe (1) arranged in proximity to the product support surface (4) configured to receive the electric signal (Se) and generate an oscillating electric field (E) permeating at least part of the food product (P) and measure a response to determine a test value (e2) indicative of a permittivity (ε) of the food product (P) at one or more frequencies (ω) in the radio wave frequency range;
- a memory (5) storing a reference value (e1) indicative of the permittivity (ε) of a reference food product (P') in a known coagulation state (U);
- a comparator (6) configured to compare the test value (e2) with the reference value (e1); and
- an evaluation module (7) configured to generate an evaluation parameter for rejecting (R) or accepting (A) the food product (P) based on the comparison.

13. The system according to claim 12, wherein the support surface (4) comprises a moving surface configured to transport the food product (P), wherein a velocity (V) of the support surface (4) is set by a transport mechanism (9), wherein the support surface (4) is configured to move the food product (P) between a first product probe (1a) configured to measure the reference value (e1) and a second product probe (1b) configured to measure the test value (e2), wherein the system (10) comprises a heater (2) for heating the food product (P), wherein a temperature (T2) of the heater (2) is set by a temperature controller (8), wherein the heater (2) is disposed in a product path between the measurement of the reference value (e1) and the measurement of the test value (e2).

14. The system according to claim 13, wherein the evaluation parameter (R,A) is used for controlling a temperature of the temperature controller (8) and/or velocity of the transport mechanism (9).

15. The system according to any of claims 12-14, comprising a discarding mechanism configured to receive the evaluation parameter (R,A) and discard the food product (P) when it is rejected (R).
